# EUROPÄISCHE PATENTSCHRIFT

(11) **EP 2 893 891 B1**
(45) Veröffentlichungstag und Bekanntmachung des Hinweises auf die Patenterteilung: **11.01.2017**
(21) Anmeldenummer: 15150799.3
(22) Anmeldetag: 27.04.2012
(51) Int. Cl.: A61B 17/86, A61B 17/70, A61B 90/00

(54) **Verschlusseinrichtung für kanülierte Knochenschrauben**
Closure device for cannulated bone screws
Dispositif de fermeture pour vis canulée pour fracture osseuse

(30) Priorität: 27.04.2011 DE 102011017602
(43) Veröffentlichungstag der Anmeldung: 15.07.2015
(62) Teilanmeldung aus: 12766903.4
(73) Patentinhaber: Silony Medical International AG, 8500 Frauenfeld (CH)
(72) Erfinder: Trautwein, Frank Thilo, 70794 Filderstadt (DE); Heuer, Frank, 70794 Filderstadt (DE); Claus, Frank, 55270 Essenheim (DE); Bernhard, Andreas, 3065 Bollingen (CH)
(74) Vertreter: DREISS Patentanwälte PartG mbB

(56) Entgegenhaltungen:
- EP-A1- 2 140 824
- WO-A1-2009/010247
- US-A1- 2007 038 219
- US-A1- 2007 293 866

## Beschreibung

Die Erfindung betrifft eine Einrichtung zum einseitigen Verschluss einer kanülierten Öffnung in einer Knochenschraube.

### Stand der Technik

Osteoporose ist durch eine Abnahme der strukturellen Integrität des Knochens gekennzeichnet. Häufig resultieren daraus Frakturen, welche zum großen Teil die Wirbelsäule betreffen. Aufgrund der reduzierten Knochendichte besteht die klinische Herausforderung in der unzureichenden Verankerungsstabilität bei der Versorgung mit Knochenschrauben.

Aus DE 3508759 A1 ist eine Knochenschraube für die Versorgung von Femurfrakturen bekannt. Die Erfindung beschreibt eine Knochenschraube mit einer mittig angeordneten Durchgangsöffnung sowie mehreren dazu querverlaufenden Öffnungen. Durch diese Öffnungen kann Knochenzement in den Knochen injiziert werden. Dadurch wird eine höhere Festigkeit zwischen Knochenschraube und Knochen erreicht.

Aus WO 01/26568 A1 und WO 2006/070961 A2 sind Knochenschrauben zur Stabilisierung der Wirbelsäule bekannt, welche eine vergleichbare Anordnung von Öffnungen für die Augmentation mit Knochenzement besitzen. Diese vorgestellten Schrauben sind am distalen Ende verschlossen, so dass ein extravertebraler Zementaustritt am distalen Schraubenende verhindert wird.

Der derzeitige Trend der medizinischen Versorgung geht in Richtung minimal-invasive Chirurgie. Eine Anforderung dieser Technik besteht in der Verwendung von geeigneten Zieldrähten mit denen die Position und Orientierung von Knochenschrauben vordefiniert werden kann. Deshalb müssen die Knochenschrauben eine zentrale Durchgangsöffnung, auch Kanülierung genannt, aufweisen, um der minimal-invasiven Technik gerecht zu werden. Werden zementierbare Knochenschrauben mit einer Durchgangsbohrung verwendet, besteht allerdings die Gefahr, dass bei einer bikortikalen Verschraubung sich der Knochenzement außerhalb vom Knochen verteilen kann.

Aus WO 00/28907 A1 ist eine kanülierte Schraube bekannt, welche einen distalen Öffnungsverschluss aufweist. Die genaue Applizierung des Verschlusses ist unbekannt und in der dargestellten Ausführung klinisch nicht oder nur schwer anzuwenden.

Aus US 2010/030135 A1 und EP 2140824 A1 sind gattungsgemäße Knochenschrauben bekannt.

### Aufgabe

Die Aufgabe der vorliegenden Erfindung besteht darin, eine Verschlusseinrichtung für zementierbare Knochenschrauben zur Verfügung zu stellen, welche besonders für die Verwendung von Führungsdrähten und damit für die minimal-invasive Technik geeignet ist. Hierbei ist das Problem zu lösen, dass Knochenzement unbeabsichtigt durch die zentrale Durchgangsbohrung der Knochenschraube austritt. Das erfindungsgemäße Implantat bzw. Implantatsystem sollte kostengünstig in der Herstellung sein da es sich regelmäßig um ein Instrument bzw. Implantat zum Einmalgebrauch handelt. Ferner soll die intra-operative Handhabung einfach sein.

### Lösung

Die Aufgabe wird erfindungsgemäß durch eine Knochenschraube mit den Merkmalen des Anspruchs 1 gelöst.

### Vorteile

Vorteilhaft bei der vorliegenden Erfindung ist die Reduktion des Herstellungsaufwands bzw. die Vereinfachung in der Handhabung. Mit der erfindungsgemäßen Verschlusseinrichtung kann die Zementapplikation bei Knochenschrauben sicherer und vor allem einfacher gestaltet werden.

### Bevorzugte Details und Ausführungsformen

Die technischen Lösungen sind nachfolgend oft beispielhaft beschrieben. Dies soll als Mittel zur Erläuterung des zugrundeliegenden Gedankens aufgefasst und nicht als auf die jeweilige konkrete Darstellung beschränkt verstanden werden.

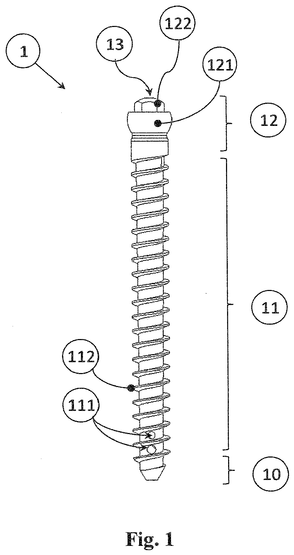

Die erfindungsgemäße Verschlusseinrichtung soll generell bei Knochenschrauben Ihre Anwendung finden. In der weiteren Beschreibung wird ein Pedikelschraubenschaft (1) als Beispiel für die unterschiedlichen Ausführungsformen der Verschlusseinrichtung dienen (Figur 1). Der Pedikelschraubenschaft (1) besteht aus einer distalen (10), einer mittleren (11) und einer proximalen Komponente (12), wobei an der proximalen Komponente ein Kopfteil (121) befestigt ist, welches dazu dient die Schraube mit einer dorsalen Implantatkomponente zu verbinden, z.B. einem Stab.

Auf die Funktion und Ausgestaltung des Schraubenschaftkopfes (12) wird in der folgenden Beschreibung nicht näher eingegangen. Ein wesentliches Merkmal des Pedikelschraubenschafts (1) ist eine zentrale Durchgangsöffnung (13). Zu dieser Durchgangsöffnung befinden sich radial angeordnete Öffnungen (111), die mit der zentralen Durchgangsöffnung (13) kommunizieren. Des Weiteren besitzt der Pedikelschraubeschaft (1) ein Knochengewinde (112).

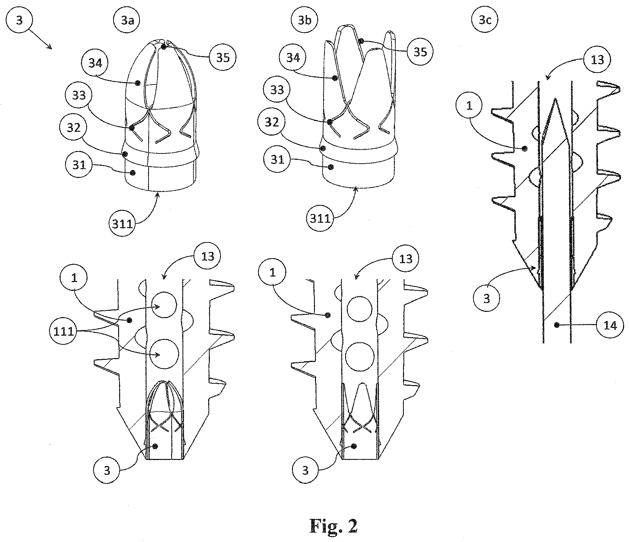

In einer Ausgestaltungsform der Erfindung kann die Verschlusseinrichtung vorzugsweise als Hülse (3) (Verschlussröhrchen) ausgeführt werden (Figuren 2). Die Hülse (3) besitzt einen distalen (31) und einen proximalen Bereich (35), wenigstens eine Zunge (34) sowie eine zentrale Durchgangsöffnung (311). Optional kann die Hülse mit einem Verankerungsbereich (32) und/oder mit einem Bereich zur Steigerung der Deformierbarkeit (33) der Zunge (34) vorgesehen werden. Die Hülse (3) befindet sich im Pedikelschraubenschaft (1) und hat die Funktion einer mechanisch einseitig wirkenden Sperre.

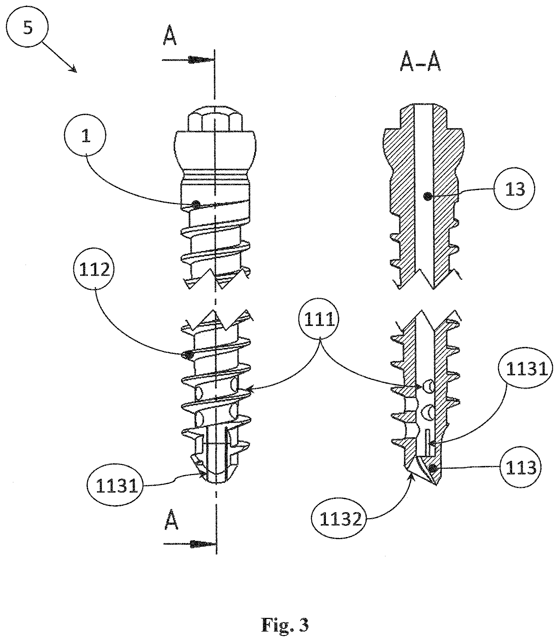

Der
Verankerungsbereich (32) dient als Fixierungseinrichtung zwischen Pedikelschraubenschaft (1) und Hülse (3). Er sichert die Hülsenposition im Pedikelschraubenschaft (1). Die Zungen (34) sind so geformt, dass sie die zentrale Durchgangsöffnung (311) im unbelasteten Zustand (3a) verschließen. Dabei sind die Zungen (34) derart angeordnet, dass sie einer axialen Belastung aus proximaler Richtung standhalten und den Zugang vom proximalen zum distalen Ende des Pedikelschraubenschafts (10) versperren. So ist das distale Pedikelschraubenende (10) bei einer Zementinjektion verschlossen. Wird eine axiale Belastung aus distaler Richtung unternommen (3b) öffnen sich die Zungen (34), und die zentrale Durchgangsöffnung (311) wird freigegeben. So kann beispielsweise ein Führungsdraht (14) durch den Pedikelschraubenschaft (1) geführt werden (Figur 3c). Das Entfernen des Führungsdrahts (14) verschließt die Hülse (3) wieder.

Die Flexibilität der Zungen (34) kann durch eine gezielte Materialandickung, -abtragung und/oder -aussparung im Bereich (33) erhöht werden.

In einer weiteren Ausgestaltungsform der Erfindung gemäß Figur 3 kann das distale Ende (10) des Pedikelschraubenschafts (1) elastisch geformt sein, um einen selbstständig schließenden Verschluss zu erzeugen. Dies kann dadurch erreicht werden, dass am distalen Ende (10) des Pedikelschraubenschafts (1) mindestens eine elastisch deformierbare Zunge (113) angebracht ist. Die Zunge (113) kann beispielsweise anhand benachbarter Schnitte (1131) realisiert werden. Optional kann eine Einkerbung (1132) am distalen Ende der Schraube (10) dazu verwendet werden, um die Einführung des Führungsdrahts (14) zu vereinfachen. Um die Anpresskraft der Zunge (113) gegenüber der Pedikelschraubenschaftinnenwandung zu erhöhen kann die Zunge (113) vorgebogen werden.

### Figurenbeschreibung

Figur 1 zeigt einen Pedikelschraubenschaft als Stellvertreter für kanülierte Knochenschrauben.
Figur 2 zeigt eine Ausführungsform mit einer Hülse mit auslenkbaren Zungen.
Figur 3 zeigt eine weitere Ausgestaltungsform einer Knochenschraube, bei welcher die Verschlusseinrichtung bereits in der Schraube vorgesehen ist.

## Patentansprüche

1. Knochenschraube mit einer zentralen Durchgangsöffnung (13), mindestens einer dazu radial verlaufenden und mit der zentralen Durchgangsöffnung kommunizierenden Öffnung (111) und einem distal befindlichen Verschluss der Durchgangsöffnung (13, 311), **dadurch gekennzeichnet, dass** der Verschluss aus mindestens einer federelastischen Zunge (34, 113) besteht, die so geformt ist, dass sie die zentrale Durchgangsöffnung (13, 311) im unbelasteten Zustand verschließt, und die derart schräg zur zentralen Durchgangsöffnung (13) der Knochenschraube angeordnet ist, dass sie einer axialen Belastung aus proximaler Richtung standhält und den Zugang vom proximalen zum distalen Ende versperrt, die zentrale Durchgangsöffnung (13, 311) jedoch bei axialer Belastung aus distaler Richtung freigibt.

2. Knochenschraube gemäß Anspruch 1, **dadurch gekennzeichnet, dass** die Zunge (34) so geformt und schräg zur zentralen Durchgangsöffnung (13) der Knochenschraube angeordnet ist, dass sie durch Einführen eines Führungsdrahtes (14) aus distaler Richtung radial ausweichen kann.

3. Knochenschraube gemäß den Ansprüchen 1 und 2, **dadurch gekennzeichnet, dass** sich die Zunge (34) an einer Hülse (3) als Träger befindet, und sich die Hülse (3) am distalen Ende der zentralen Durchgangsöffnung (13) der Knochenschraube befindet.

## Claims

1. A bone screw having a central through opening (13), at least one opening (111) extending radially thereto and communicating with the central through opening, and a distally located closure of the through opening (13, 311), **characterized in that** the closure comprising at least one spring-elastic tongue (34, 113), which is shaped such that in the unstressed state it closes the central through opening (13, 311), and which is located obliquely to the central through opening (13) of the bone screw in such a way that it withstands an axial stress from the proximal direction and blocks the access from the proximal to the distal end, but upon axial stress from the distal direction it uncovers the central through opening (13, 311).

2. The bone screw of claim 1, **characterized in that** the tongue (34) is shaped and located obliquely to the central through opening (13) of the bone screw in such a way that it can swerve radially as a result of the introduction of a guide wire (14) from the distal direction.

3. The bone screw of claims 1 and 2, **characterized in that** the tongue (34) is located on a sheath (3) as a substrate, and the sheath (3) is located on the distal end of the central through opening (13) of the bone screw.

## Revendications

1. Vis à os ayant une ouverture centrale de passage (13), au moins une ouverture (111) s'étendant radialement par rapport à celle-ci et communiquant avec ladite ouverture centrale de passage, ainsi qu'un obturateur de l'ouverture de passage (13, 311) qui est situé côté distal, **caractérisée par le fait que** ledit obturateur se compose d'au moins une languette (34, 113) à élasticité de ressort qui est formée de telle sorte qu'elle ferme ladite ouverture centrale de passage (13, 311) à l'état non chargé, et qui est disposée obliquement par rapport à l'ouverture centrale de passage (13) de la vis à os de telle manière qu'elle résiste à une charge axiale provenant de la direction proximale et obstrue l'accès depuis l'extrémité proximale à l'extrémité distale, mais qu'elle libère l'ouverture centrale de passage (13, 311) dans le cas d'une charge axiale provenant de la direction distale.

2. Vis à os selon la revendication 1, **caractérisée par le fait que** ladite languette (34) est formée et disposée obliquement par rapport à l'ouverture centrale de passage (13) de la vis à os de telle sorte qu'elle peut se ranger radialement en introduisant un fil de guidage (14) depuis la direction distale.

3. Vis à os selon les revendications 1 et 2, **caractérisée par le fait que** ladite languette (34) se trouve sur une douille (3) en tant que support et que ladite douille (3) est située à l'extrémité distale de l'ouverture centrale de passage (13) de la vis à os.
